(19) Europäisches Patentamt

European Patent Office

Office européen des brevets

(11) **EP 1 159 950 A1**

(12) **DEMANDE DE BREVET EUROPEEN**

(43) Date de publication:
**05.12.2001 Bulletin 2001/49**

(51) Int Cl.[7]: **A61K 7/02**, A61K 7/021, A61K 7/027

(21) Numéro de dépôt: **01117660.9**

(22) Date de dépôt: **06.08.1999**

(84) Etats contractants désignés:
**AT BE CH CY DE DK ES FI FR GB GR IE IT LI LU MC NL PT SE**

(30) Priorité: **10.08.1998 FR 9810255**

(62) Numéro(s) de document de la (des) demande(s) initiale(s) en application de l'article 76 CBE:
**99401999.0 / 0 979 643**

(71) Demandeur: **L'OREAL**
**75008 Paris (FR)**

(72) Inventeurs:
• **Arnaud, Pascal**
**94240 L'Hay Les Roses (FR)**

• **Auguste, Frédéric**
**94550 Chevilly-Larue (FR)**

(74) Mandataire: **Casalonga, Axel et al**
**BUREAU D.A. CASALONGA - JOSSE**
**Morassistrasse 8**
**80469 München (DE)**

Remarques:
Cette demande a été déposée le 25 - 07 - 2001 comme demande divisionnaire de la demande mentionnée sous le code INID 62.

(54) **Composition de maquillage ou de soin sans transfert**

(57) L'invention concerne une composition de maquillage ou de soin sans transfert renfermant l'association

- d'une huile hydrocarbonée volatile et
- d'au moins une cire synthétique portant au moins une fonction hydroxyle ou carboxyle et ayant un point de fusion compris entre 75 °C et 120 °C et des paramètres de solubilité de Hansen ($\delta_d$, $\delta_p$, et $\delta_h$) tels que :

$$15,50 \leq \delta_d \leq 18,50 \ J^{1/2}/cm^{3/2}$$

et

$$4,50 \leq \delta_a \leq 7,50 \ J^{1/2}/cm^{3/2}$$

avec

$$\delta_a = (\delta_p{}^2 + \delta_h{}^2)^{1/2}.$$

# EP 1 159 950 A1

**Description**

La présente invention concerne une composition de maquillage ou de soin de la peau, des fibres kératiniques (cils, sourcils ou cheveux), ou des lèvres, sans transfert renfermant l'association d'une huile hydrocarbonée volatile et d'une cire synthétique fonctionnalisée, l'utilisation de cette association pour conférer des propriétés non-transfert à des compositions de maquillage ou de soin de la peau, des fibres kératiniques ou des lèvres, ainsi qu'un procédé de fabrication de telles compositions de maquillageou de soin sans transfert.

La mise au point de produits de maquillage ou de soin dits "sans transfert" fait actuellement l'objet de nombreuses recherches cosmétologiques. Ces produits, par exemple des fonds de teint ou des rouges à lèvres, des fards à paupières ou à joues, se distinguent par le fait qu'une fois appliqués sur la peau ou les lèvres, ils ne se déposent pas de façon notable sur les surfaces avec lesquelles ils viennent en contact (verre, tasse, cigarette, vêtement par exemple).

Une première approche pour empêcher le transfert des produits cosmétiques appliqués a consisté à les recouvrir d'une couche de produits réputés pour leur propriétés anti-adhésives, tels que des produits fluorés ou siliconés. Les formulations de ce type présentent cependant l'inconvénient d'une mauvaise cosméticité. Par exemple, le film de rouge à lèvres devient huileux et susceptible de migrer sur la peau contiguë aux lèvres et aux paupières.

Une autre possibilité pour obtenir des produits "sans transfert" consiste à utiliser des polymères ou résines siliconées en association avec des matières premières volatiles qui laissent, après évaporation de ces dernières, un film inerte résistant au transfert sur d'autres surfaces. Les matières premières volatiles utilisées sont par exemple des silicones cycliques de très faible viscosité (inférieure à 3 centistokes) ou encore des isoparaffines.

Pour que ces produits de maquillage se présentent sous forme de solide, il est nécessaire d'y adjoindre des composés de durcissement tels que des cires. Se posent alors des problèmes de stabilité mécanique et/ou de compatibilité entre les cires et les produits volatils. On constate en effet, pour une faible teneur en cires, une dureté insuffisante du stick qui peut être à l'origine de problèmes de stabilité ou d'utilisation. La simple augmentation de la proportion de cires durcissantes ne permet pas de résoudre ces problèmes car elle se traduit généralement par une dégradation des propriétés cosmétiques du produit qui devient inconfortable à porter.

L'objectif de la présente invention a été de trouver des cires permettant un durcissement suffisant, c'est-à-dire ayant un pouvoir de gélification maximal, de manière à limiter la proportion de ces cires dans les produits cosmétiques solides sans transfert et d'éviter les problèmes d'incompatibilité.

Le problème du transfert existe aussi pour les produits de soin ou de traitement de la peau, contenant des actifs, colorés ou non.

La demanderesse a trouvé de manière inattendue qu'une catégorie particulière de cires hydrocarbonées caractérisées par un domaine de fusion et par des paramètres de solubilité particuliers, permettaient de résoudre les problèmes précédemment évoqués.

L'utilisation de telles cires pour la gélification de véhicules siliconés liquides volatiles tels que les cyclométhicones en vue de la préparation de déodorants-crèmes est décrite dans le document WO 97/17942, mais ce document ne concerne nullement l'obtention de produit solide sans transfert à base d'isoparaffines.

La présente invention a donc pour objet une composition cosmétique de maquillage ou de soin de la peau, des lèvres ou des fibres kératiniques sans transfert comprenant au moins une huile hydrocarbonée volatile et au moins une cire synthétique fonctionnalisée remplissant les critères de température de fusion et de paramètres de solubilité bien particuliers définis ci-après.

L'invention a également pour objet l'utilisation des cires synthétiques remplissant les critères de température de fusion et de paramètres de solubilité indiqués ci-dessous pour le durcissement de compositions de maquillage ou de soin de la peau, des lèvres et des fibres kératiniques renfermant des huiles hydrocarbonées volatiles, dans le but d'obtenir des compositions cosmétiques solides "sans transfert".

Encore un autre objet de la présente invention est un procédé de préparation de compositions de maquillage ou de soin solides "sans transfert" associant des huiles hydrocarbonées volatiles et des cires synthétiques ayant les propriétés ci-dessous.

La présente invention a aussi pour objet un procédé pour limiter le transfert d'une composition de maquillage ou de soin de la peau, des lèvres ou des fibres kératiniques sur une surface avec laquelle, la peau, les lèvres ou les fibres kératiniques sont mise en contact, consistant à introduire dans la composition l'association des huiles hydrocarbonées et de cires synthétiques telles que définies ci-dessous.

Les compositions cosmétiques solides "sans transfert" de la présente invention comprennent donc, dans une phase grasse,

- au moins une huile hydrocarbonée volatile et
- au moins une cire synthétique fonctionnalisées, c'est-à-dire portant au moins une fonction hydroxyle ou carboxyle, et ayant un point de fusion compris entre 75 °C et 120 °C et des paramètres de solubilité de Hansen ($\delta_d$, $\delta_p$, et

2

$\delta_h$) tels que :

$$15{,}50 \leq \delta_d \leq 18{,}50 \; J^{1/2}/cm^{3/2}$$

et

$$4{,}50 \leq \delta_a \leq 7{,}50 \; J^{1/2}/cm^{3/2}$$

avec

$$\delta_a = (\delta_p{}^2 + \delta_h{}^2)^{1/2}.$$

On entend par huile volatile dans la présente invention, une huile susceptible de s'évaporer à température ambiante d'un support sur lequel elle a été appliquée, autrement dit une huile ayant une tension de vapeur mesurable à température ambiante, et par huile hydrocarbonée volatile une huile dont le squelette ne contient que des hydrogènes et des carbones.

Les huiles hydrocarbonées volatiles préférées convenant dans la présente invention sont en particulier les isoparaffines, à savoir des alcanes ramifiés, comportant de 8 à 16 atomes de carbone. On peut bien entendu également utiliser des mélanges de telles isoparaffines.

L'huile volatile hydrocarbonée utilisée de préférence pour la présente invention est l'isododécane, c'est-à-dire le 2,2,4,4,6-pentaméthylheptane.

On peut citer à titre d'exemple d'isododécane disponible sur le marché le produit commercialisé sous la dénomination PERMETHYL® 99A par la société PRESPERSE Inc.

Les cires synthétiques fonctionalisées utilisées pour la gélification et le durcissement des compositions de maquillage de la présente invention comportent au moins un groupe hydroxyle ou carboxyle et sont définies par leur température de fusion et par leur paramètres de solubilité dans l'espace tridimensionnel de solubilité selon Hansen (*The three-dimensional solubility parameters*, J. Paint Technol., 39, page 105 (1967)).

Les cires synthétiques présentent l'avantage d'être des produits de composition constante et avoir une teneur en alcool gras ou acide gras constante, à l'inverse des cires d'origine naturelle.

Selon Hansen, le changement d'énergie interne lors de la vaporisation est considéré comme étant la somme de trois contributions, la première due aux liaisons hydrogène (indice h), la seconde aux interactions dipolaires (indice p) et la troisième aux forces de dispersion (indice d).

Le paramètre de solubilité $\delta$ (défini comme la racine carrée de la variation d'énergie interne par unité de volume de l'espèce chimique lors de sa vaporisation et exprimé en $(J/cm^3)^{1/2}$ est défini comme un vecteur de coordonnées ($\delta_h$, $\delta_p$ et $\delta_d$), l'amplitude du vecteur étant donnée par

$$\delta^2 = \delta_h{}^2 + \delta_p{}^2 + \delta_d{}^2$$

Le paramètre $\delta_a$ utilisé dans la présente invention comme critère de sélection des cires appropriées est défini par l'équation suivante :

$$\delta_a = (\delta_p{}^2 + \delta_h{}^2)^{1/2}$$

Le pouvoir gélifiant des cires synthétiques de la présente invention est lié aux phénomènes de compatibilité/incompatibilité de celles-ci avec la phase grasse, notamment avec les isoalcanes.

Des cires ayant un paramètre $\delta_a$ inférieur à 4,5 présentent une trop grande compatibilité avec les isoalcanes et ont par conséquent un pouvoir gélifiant et structurant insuffisant. D'autre part, un paramètre $\delta_a$ supérieur à 7,5 est le signe d'une trop forte incompatibilité entre la cire et les isoalcanes qui se traduit par des phénomènes de démixion, d'inhomogénéité et éventuellement par un manque de stabilité mécanique.

Dans un mode de réalisation préféré de la présente invention, les cires remplissant ces critères de sélection sont les alcools gras linéaires à très longue chaîne correspondant à la formule chimique suivante

$$(1) \quad CH_3\text{-}(CH_2)_n\text{-}CH_2OH$$

dans laquelle n est un entier variant de 18 à 58.

De tels alcools gras en $C_{20-60}$ sont disponibles dans le commerce par exemple auprès de la société NEW PHASE TECHNOLOGIES sous les dénominations PERFORMACOL® 350, PERFORMACOL® 425, PERFORMACOL® 550 et PERFORMACOL® 700, ou par la société PETROLITE sous les dénominations UNILIN® 350 Alcohol, UNILIN® 425 Alcohol, UNILIN® 550 Alcohol et UNILIN® 700 Alcohol. Il s'agit de mélanges d'alcools linéaires à très longue chaîne obtenus par un procédé de polymérisation permettant d'obtenir des polymères à très faible indice de polydispersité ($M_p/M_n \approx 1,1$). Leur masse molaire moyenne en poids est comprise entre environ 350 et 1000.

Dans les compositions de maquillage sans transfert de la présente invention, l'huile hydrocarbonée volatile représente généralement de 5 à 90 %, de préférence de 5 à 80 % et mieux de 10 à 60% en poids de la composition cosmétique totale. Ces huiles hydrocarbonées peuvent représenter 100% de la phase volatile présente dans la composition.

La ou les cires synthétiques fonctionnalisées représentent généralement de 5 % à 30 %, de préférence de 8 % à 20 % en poids de la composition totale.

La phase grasse des compositions cosmétiques de la présente invention peut contenir, outre les huiles hydrocarbonées volatiles, en particulier les isoparaffines en $C_{8-16}$, et les cires synthétiques fonctionnalisées définies précédemment, une ou plusieurs cires d'origine animale, végétale ou synthétique différentes des alcools gras et acides gras en $C_{20-60}$ décrits ci-dessus, ayant de préférence un point de fusion supérieur à 30°C et idéalement supérieur à 45°C. Ces cires sont choisies, entre autres, parmi la lanoline éventuellement hydrogénée, hydroxylée ou acétylée, la cire d'abeille, le spermacéti, les alcools de lanoline, les acides gras de lanoline et l'alcool de lanoline acétylée, la cire de Carnauba, de Candellila, de kapok, d'Ouricury, de riz, de jojoba hydrogénée, d'Alfa ou du Japon, les cires de fibres de liège ou de canne à sucre, le beurre de cacao, les cires de paraffine, de lignite, de pétrolatum, de vaseline ou les cires microcristallines, la cérésine, l'ozokérite, la cire de montan, les cires de polyéthylène, les cires obtenues par synthèse de Fischer-Tropsch, les esters linéaires résultant de la réaction d'un acide carboxylique saturé en $C_{10-40}$ et d'un alcool saturé en $C_{10-40}$, l'alcool cétylique, l'alcool stéarylique, les lanolates ou stéarates de calcium, les huiles de ricin, de palme, de coco, de tournesol ou de coprah hydrogénées.

Elles peuvent contenir également un ou plusieurs solvants volatils supplémentaires, différents des huiles hydrocarbonées volatiles de la présente invention. On peut citer à titre d'exemple de ces solvants volatils supplémentaires les huiles de silicones cycliques et volatiles telles que les cyclométhicones($D_4$, $D_5$, $D_6$), les silicones linéaires volatiles telles que le décaméthyltétrasiloxane ($L_4$), l'octylheptaméthyltrisiloxane, l'octaméthyltrisiloxane ($L_3$), l'hexyl-heptaméthyltrisiloxane, et les huiles volatiles fluorées telles que le nonafluorométhyoxybutane ou le perfluorométhylcyclopentane.

Ces solvants volatils représentent de préférence de 0 à 50% en poids de la phase volatile.

Les compositions de la présente invention peuvent contenir également des huiles siliconées et/ou hydrocarbonées et/ou fluorées non volatiles, des gommes de silicone et des cires de silicone.

Les huiles de silicone non volatiles utilisables dans la composition selon l'invention peuvent être des huiles de faible viscosité telles que les polysiloxanes linéaires, dont le degré de polymérisation est de préférence de 6 à 2000 environ. On peut citer, par exemple: les polydiméthylsiloxanes (PDMS) de viscosité supérieure à 10 mPa.s, les phényldiméthicones, les phényltriméthicones, les polyphénylméthylsiloxanes et leurs mélanges.

Les gommes de silicone utilisables dans le produit de l'invention peuvent être des polysiloxanes de masse moléculaire élevée, de l'ordre de 200 000 a 1 000 000 et ayant une viscosité supérieure à 500 000 mPa.s. Elles peuvent être utilisées seules ou en mélange avec un solvant tel qu'une huile polydiméthylsiloxane ou polyphénylsiloxane.

Les gommes peuvent être présentes a raison de 0 a 2% en poids de matière active dans le produit coule final, de préférence a raison de 0,1 a 1%.

Les cires de silicone utilisable dans la composition selon l'invention peuvent être des polysiloxanes linéaires substitués. On peut citer, par exemple, les cires de silicone polyéther, les alkyl ou alkoxydiméthicones ayant de 16 a 45 atomes de carbone. Ces cires de silicone peuvent être présentes à raison de 0 à 18% en poids dans la composition finale, de préférence à raison de 2 a 15%.

Les huiles hydrocarbonées utilisables dans la composition selon l'invention peuvent être des huiles d'origine végétale, animale, minérale ou synthétique.

Comme huiles hydrocarbonées non volatiles utilisables dans l'invention, on peut citer notamment:

- les huiles hydrocarbonées d'origine animale telles que le perhydrosqualène;
- les huiles hydrocarbonées végétales telles que les triglycérides liquides d'acides gras de 4 à 10 atomes de carbone comme les triglycerides des acides heptanoïque ou octanoïque, ou encore les huiles de tournesol, de maïs, de soja, de courge, de pépins de raisin, de sésame, de noisette, d'abricot, de macadamia, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, de beurre de karité;

- les hydrocarbures linéaires ou ramifiés, d'origine minérale ou synthétique tels que la vaseline, les polydécènes, le polyisobutène hydrogéné tel que le parléam;

- les esters et les éthers de synthèse comme les huiles de formule $R_1COOR_2$ dans laquelle $R_1$ représente le reste d'un acide gras supérieur comportant de 6 à 29 atomes de carbone et $R_2$ représente une chaîne hydrocarbonée contenant de 3 à 30 atomes de carbone, telles que l'huile de Purcellin, le myristate d'isopropyle, le palmitate d'éthyl-2 hexyle, le stéarate d'octyl-2 dodécyle, l'érucate d'octyl-2 dodécyle, l'isostéarate d'isostéaryle, le propionate d'arachidyle, le benzoate d'octyl-2 dodecyle; les esters hydroxylés comme l'isostéaryl lactate, l'octylhydroxystéarate, l'hydroxystéarate d'octyldodécyle, le diisostéarylmalate, le citrate de triisocétyle ; les esters de polyol comme le dioctanoate de propylène glycol, le diheptanoate de néopentylglycol, le diisononanoate de diéthylèneglycol et les esters du pentaérythritol ;

- les alcools gras liquides à température ambiante à chaîne carbonée ramifiée et/ou insaturée ayant de 12 à 24 atomes de carbonne comme l'octyldodécanol, le 2-hexyldécanol, le 2-butyloctanol, le 2-undécylantadécanol, l'alcool isostéarylique oléique ; leurs mélanges.

Ces huiles non volatiles sont présentes de préférence en une quantité allant de 5 à 60 % en poids de la composition totale.

Les compositions cosmétiques sans transfert peuvent contenir bien entendu également les principes actifs qui leur confèrent leur propriétés cosmétiques caractéristiques et des adjuvants cosmétiques. Il s'agit par exemple de substances telles que les filtres solaires, les agents anti-radicaux libres, les agents hydratants, les vitamines, les protéines, les céramides, les agents régulateurs de pH, les anti-oxydants, les agents conservateurs, les charges, les pigments et colorants, les émollients, les agents anti-mousse, les parfums, les agents tensio-actifs et les plastifiants.

Bien entendu, l'homme de métier veillera à choisir ce ou ces éventuels composés complémentaires et leur quantité de manière à ce que les propriétés avantageuses attachées intrinsèquement à la composition cosmétique de l'invention ne soient pas, ou substantiellement pas, altérées par la ou les adjonctions envisagées.

La composition de l'invention contient avantageusement une phase particulaire généralement présente à raison de 0,05 a 35 % du poids total de la composition, de préférence de 2 à 25 %, et qui peut comprendre des pigments et/ou des nacres et/ou des charges habituellement utilisés dans les compositions cosmétiques. Cette charge peut conduire à une composition colorée, blanche ou incolore.

Par pigments, il faut comprendre des particules blanches ou colorées, minérales ou organiques, insolubles dans la phase grasse liquide, destinées à colorer et/ou opacifier le produit coulé. Par charges, il faut comprendre des particules incolores ou blanches, minérales ou de synthèse, lamellaires ou non lamellaires. Par nacres, il faut comprendre des particules irisées, notamment produites par certains mollusques dans leur coquille ou bien synthétisées. Ces charges et nacres servent notamment à modifier la texture de la composition.

Les pigments peuvent être présents dans la composition à raison de 0,05 à 25 % du poids de composition finale, et de préférence a raison de 2 à 15 %. Comme pigments minéraux utilisables dans l'invention, on peut citer les oxydes de titane, de zirconium ou de cérium ainsi que les oxydes de zinc, de fer ou de chrome et le bleu ferrique. Parmi les pigments organiques utilisables dans l'invention, on peut citer le noir de carbone, et les laques de baryum, strontium, calcium (DC Red N°7), aluminium.

Les nacres peuvent être présentes dans la composition à raison de 0 à 20 % du poids total du produit coulé, de préférence à un taux de l'ordre de 1 à 15 %. Parmi les nacres utilisables dans l'invention, on peut citer le mica recouvert d'oxyde de titane, d'oxyde de fer, de pigment naturel ou d'oxychlorure de bismuth tel que le mica titane coloré

Les charges peuvent être présentes à raison de 0 à 35 % du poids total de la composition, de préférence 2 a 15 %. On peut notamment citer le talc, le mica, la silice, le kaolin, les poudres de Nylon (Orgasol notamment) et de polyéthylène, le Téflon, l'amidon, le nitrure de bore, des microsphères de copolymères telles que l'Expancel (Nobel Industrie), le polytrap (Dow Corning) et les microbilles de résine de silicone (Tospearl de Toshiba, par exemple).

La composition peut également comprendre un tensio-actif, par exemple un tensio-actif usuel anionique ou non ionique. Le tensio-actif est de préférence présent, à raison de 0,5 à 8% en poids de la composition.

Elle peut également comprendre des colorants liposolubles et/ou des colorants hydrosolubles.

Les compositions cosmétiques de l'invention peuvent se présenter sous une forme solide, pâteuse, ne s'écoulant pas sous son propre poids. Il peut s'agir d'émulsions ou de compositions anhydres.

La forme mettant le mieux à profit les propriétés de durcissement de la catégorie de cires utilisée dans la présente invention est bien entendu la forme stick ou coulée en coupelle. Elle constitue par conséquent un mode de réalisation préféré de la présente invention.

Les procédés de fabrication des produits de maquillage ou de soin selon l'invention ne diffèrent en rien des procédés classiquement utilisés en cosmétique et sont parfaitement connus de l'homme de l'art.

Un produit de maquillage solide coulé tel qu'un rouge à lèvres, un fond de teint solide, un produit anti-cernes ou encore un produit de coloration ou de protection de la peau ou un mascara "pain" ou une poudre compacte, sont fabriqués par exemple par

- fusion et mélange des composants non volatils de la composition,
- addition, à une température plus basse, de la phase volatile,
- coulage du mélange ainsi obtenu dans un moule de forme appropriée, et
- refroidissement jusqu'à température ambiante.

L'invention est illustrée plus en détail dans les exemples suivants.

**Exemple 1**

Evaluation de la dureté d'une composition renfermant l'association isododécane + cire

On prépare différents sticks à partir d'une composition type constituée de 85 % en poids d'isododécane (PERMETHYL® 99A de la société PRESPERSE) et de 15 % d'une cire dont la nature chimique varie d'un stick à l'autre. Les sticks sont fabriqués par fusion et mélange de ces deux composants, coulage dans un moule cylindrique approprié et refroidissement. La dureté des sticks obtenus est mesurée à 20°C au moyen d'un dynamomètre DFGHS 2 de la société INDELCO-CHATILLON se déplaçant à une vitesse de 100 mm/minute. Elle est exprimée comme la force de cisaillement (exprimée en grammes) nécessaire pour couper un stick de 12,7 mm de diamètre dans ces conditions.
Le Tableau 1 ci-dessous présente les différentes cires utilisées, leur température de fusion, les paramètres de solubilité $\delta_d$ et $\delta_a$ ainsi que les résultats des essais de dureté obtenus.

Tableau 1

| cire | $T_{fusion}$ (°C) | $\delta_d$ (J$^{1/2}$ cm$^{-3/2}$) | $\delta_a$ (J$^{1/2}$ cm$^{-3/2}$) | force de cisaillement (g) |
|---|---|---|---|---|
| cire de polyéthylène PERFORMALENE 500 (New Phase Technologies) | 86 | 16,29 | 0 | 6 |
| cire de polyéthylène PERFORMALENE655 (New Phase Technologies) | 102 | 16,35 | 0 | 6 |
| stéarate d'octanosyle KESTER WAX 82H (Koster Keunen) | 82 | 16,52 | 3,07 | 4 |
| alcool gras linéaire PERFORMACOL700 (New Phase Technologies) | 105 | 16,58 | 4,62 | 73 |
| alcool gras linéaire PERFORMACOL550 (New Phase Technologies) | 99 | 16,54 | 4,73 | 75 |
| alcool gras linéaire PERFORMACOL425 (New Phase Technologies) | 91 | 16,51 | 5,55 | 28 |
| hydroxystéarate d'hydroxy octacosanyle ELFACOS C26 (Akzo Nobel) | 80 | 16,75 | 7,9 | 1 |
| huile de ricin hydrogénée CUTINAHR (Henkel) | 86 | 16,96 | 9,01 | non compatible |

Ces résultats des essais de dureté montrent que seules les cires selon la présente invention, à savoir celles ayant un paramètre $\delta_a$ compris entre 4,5 et 7,5 J$^{1/2}$/cm$^{3/2}$, permettent d'obtenir des sticks d'une dureté suffisante. Les autres cires, bien qu'ayant une température de fusion et un paramètre $\delta_d$ situés à l'intérieur des intervalles définis dans la présente invention, conduisent à des sticks trop mous ou présentant des problèmes d'incompatibilité.

**Exemple 2**

On a préparé de la manière indiquée dans l'Exemple 1 différents sticks à partir de la composition type suivante :

| | |
|---|---|
| cire (voir Tableau 2) | 16 g |
| isododécane | 38,8 g |
| (PERMETHYL® 99A, Presperse) ozokérite | 6,4 g |
| (OZOKERITE WAX SP 1020, Strahl Pitsch) silicone volatile | 38,8 g |
| (DC 200 fluide 1,5 cSt, Dow Corning) | 100 g |

La dureté des sticks est mesurée à 20°C au moyen d'un analyseur de texture TA-XT2 commercialisé par la société RHEO.

La dureté est assimilée à la force maximale (en Newton) mesurée lors de la pénétration d'un cylindre en inox de 5 mm de diamètre sur une profondeur de 0,3 mm et à une vitesse de 0,1 mm/seconde à l'intérieur d'un stick à tester.

Les résultats obtenus sont rassemblés dans le Tableau 2 ci-après.

Tableau 2

| cire | $T_f$ (°C) | $\delta_d$ (J$^{1/2}$ cm$^{-3/2}$) | $\delta_a$ (J$^{1/2}$ cm$^{-3/2}$) | F max (N) |
|---|---|---|---|---|
| cire de polyéthylène PERFORMALENE® 500 (New Phase Technologies) | 86 | 16,29 | 0 | 0,17 |
| cire de polyéthylène A-C 1702 (Allied Chemical) | 92 | 16,3 | 0 | non mesurable trop mou < 0,17 |
| alcool gras linéaire PERFORMACOL® 550 (New Phase Technologies) | 99 | 16,54 | 4,73 | 3,23 |

Ces résultats montrent que les cires synthétiques selon la présente invention permettent de durcir de manière satisfaisante une composition complexe susceptible de constituer la base d'un produit de maquillage sans transfert, alors que des cires non conformes à l'invention car ayant un paramètre de solubilité $\delta_a$ non compris dans l'intervalle défini précédemment, aboutissent à des compositions d'une tenue mécanique insatisfaisante.

**Exemple 3**

Rouges à lèvres

On prépare les rouges à lèvres sans transfert A, B, C en stick suivants :

| | A | B comparatif | C comparatif |
|---|---|---|---|
| alcool gras linéaire PERFORMACOL® 550 (New Phase Technologies) | 10,00 | - | - |
| cire de polyéthylène PERFORMALENE® 500 (New Phase Technologies) | | 10,00 | 23,00 |
| isododécane | 40,00 | 40,00 | 33,61 |
| cycopentapolysiloxane | 10,00 | 10,00 | 8,39 |
| polyisobutène hydrogéné | 31,34 | 31,34 | 26,34 |
| DC Red n° 7 Calcium lake | 2,90 | 2,90 | 2,90 |
| oxyde de titane | 1,80 | 1,80 | 1,80 |
| FD et C yellow n°6 Aluminium lake | 3,30 | 3,30 | 3,30 |
| oxyde de fer noir | 0,06 | 0,06 | 0,06 |
| DC Red n° 21 Aluminium lake | 0,60 | 0,60 | 0,60 |
| | 100 % en poids | 100 % en poids | 100 % en poids |
| force de cisaillement (en grammes) | 70 | 8 | 75 |

La comparaison des compositions A et B montre que, pour une teneur en cire donnée, seul l'alcool gras PERFORMACOL® 550 conforme à l'invention donne un rouge à lèvres ayant une dureté satisfaisante.

Pour atteindre une dureté comparable avec une cire non conforme à l'invention, il est nécessaire d'augmenter considérablement la teneur en cire (composition C). L'augmentation de la teneur en cire se traduit cependant par une détérioration des propriétés cosmétiques du stick de rouge à lèvres. En effet, la composition A est considérablement plus glissante, moins sèche et plus homogène que la composition C.

**Revendications**

1.   Composition de maquillage ou de soin sans transfert, **caractérisée par le fait qu'**elle comprend, dans un milieu physiologiquement acceptable l'association,

- d'au moins une huile hydrocarbonée volatile constituée uniquement d'atomes de carbone et d'atomes d'hydrogène, et
- d'au moins une cire synthétique fonctionnalisée portant au moins une fonction hydroxyle ou carboxyle et ayant un point de fusion compris entre 75 °C et 120 °C et des paramètres de solubilité de Hansen ($\delta_d$, $\delta_p$, et $\delta_h$) tels que :

$$15,50 \leq \delta_d \leq 18,50 \ J^{1/2}/cm^{3/2}$$

et

$$4,50 \leq \delta_a \leq 7,50 \ J^{1/2}/cm^{3/2}$$

avec

$$\delta_a = (\delta_p{}^2 + \delta_h{}^2)^{1/2}$$

**2.** Composition sans transfert selon la revendication 1, **caractérisée en ce que** l'huile hydrocarbonée volatile est une isoparaffine en $C_{8-16}$ ou un mélange d'isoparaffines en $C_{8-16}$.

**3.** Composition sans transfert selon la revendication 2, **caractérisée en ce que** l'huile hydrocarbonée volatile est l'isododécane (2,2,4,4,6-pentaméthylheptane).

**4.** Composition sans transfert selon la revendication 1, **caractérisée en ce que** la cire synthétique fonctionnalisée est un alcool gras linéaire en $C_{20-60}$.

**5.** Composition sans transfert selon l'une quelconque des revendications précédentes, **caractérisée en ce que** l'huile hydrocarbonée volatile représente de *5* à 90 %, de préférence de *5* à 80 % et mieux de 10 à 60% en poids de la composition totale.

**6.** Composition sans transfert selon l'une quelconque des revendications précédentes, **caractérisée en ce que** la ou les cires synthétiques fonctionnalisées représentent de 5 à 30 %, de préférence de 8 à 20 % en poids de la composition totale.

**7.** Composition sans transfert selon l'une quelconque des revendications précédentes, **caractérisée en ce qu'**elle comprend en outre une ou plusieurs cires d'origine animale, végétale ou synthétique différentes des cires synthétiques fonctionalisées.

**8.** Composition sans transfert selon l'une quelconque des revendications précédentes **caractérisée en ce qu'**elle comprend en outre un ou plusieurs solvants volatils différents des huiles hydrocarbonées volatiles.

**9.** Composition sans transfert selon l'une quelconque des revendications précédentes, **caractérisée en ce qu'**elle comprend en outre une gomme de silicone, de préférence en une quantité au plus égale à 2 % en poids.

**10.** Composition sans transfert selon l'une quelconque des revendications précédentes, **caractérisée en ce qu'**elle comprend en outre au moins une huile non volatile d'origine végétale, animale, minérale ou synthétique, de préférence en une quantité allant de 5 à 60 % en poids de la composition totale.

**11.** Composition sans transfert selon l'une quelconque des revendications précédentes, **caractérisée en ce qu'**elle comprend en outre un ou plusieurs adjuvants ou principes actifs cosmétiques choisis parmi les filtres solaires, les agents anti-radicaux libres, les agents hydratants, les vitamines, les protéines, les céramides, les agents régulateurs de pH, les anti-oxydants, les agents conservateurs, les charges, les pigments et colorants, les émollients, les agents anti-mousse, les parfums, les agents tensioactifs et les plastifiants.

**12.** Composition sans transfert selon l'une quelconque des revendications précédentes, **caractérisée en ce qu'**elle se présente sous la forme d'un produit de maquillage ou de soin solide ou pâteux, anhydre ou en émulsion.

**13.** Composition sans transfert selon la revendication 12, **caractérisée en ce que** le produit de maquillage est un rouge à lèvres, un fond de teint, un fard à paupières ou à joues ou un mascara.

**14.** Composition de maquillage sans transfert selon la revendication 12, **caractérisée en ce que** le produit de maquillage solide est un stick de rouge à lèvres, un fond de teint ou une poudre compacte.

**15.** Utilisation de l'association d'au moins une huile hydrocarbonée volatile constituée uniquement d'atomes de carbone et d'atomes d'hydrogène, et d'au moins une cire synthétique fonctionnalisée portant au moins un groupe hydroxyle ou carboxyle et ayant un point de fusion compris entre 75 °C et 120 °C et des paramètres de solubilité de Hansen ($\delta_d$, $\delta_p$, et $\delta_h$) tels que :

$$15,50 \leq \delta_d \leq 18,50 \ J^{1/2}/cm^{3/2}$$

et

$$4{,}50 \leq \delta_a \leq 7{,}50 \ J^{1/2}/cm^{3/2}$$

avec

$$\delta_a = (\delta_p{}^2 + \delta_h{}^2)^{1/2}$$

pour la fabrication d'un produit cosmétique de maquillage ou de soin sans transfert.

16. Procédé pour limiter le transfert d'une composition de soin ou de maquillage de la peau, des lèvres, ou des fibres kératiniques sur une surface avec laquelle, la peau, les lèvres ou les fibres sont mises en contact consistant à introduire dans la composition l'association telle que définie dans les revendications 1 à 6.

## RAPPORT PARTIEL DE RECHERCHE EUROPEENNE

**Office européen des brevets**

qui selon la règle 45 de la Convention sur le brevet européen est considéré, aux fins de la procédure ultérieure, comme le rapport de la recherche européenne

**Numéro de la demande**

EP 01 11 7660

### DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| A | EP 0 850 644 A (L'OREAL)<br>1 juillet 1998 (1998-07-01)<br>* le document en entier * | 1-16 | A61K7/02<br>A61K7/021<br>A61K7/027 |
| A | EP 0 847 752 A (L'OREAL)<br>17 juin 1998 (1998-06-17)<br>* le document en entier * | 1-16 | |
| A | WO 96 40044 A (PROCTER & GAMBLE)<br>19 décembre 1996 (1996-12-19)<br>* le document en entier * | 1-16 | |
| A | WO 96 15761 A (PROCTER & GAMBLE)<br>30 mai 1996 (1996-05-30)<br>* le document en entier * | 1-16 | |
| A | EP 0 709 083 A (REVLON CONSUMER PRODUCTS)<br>1 mai 1996 (1996-05-01)<br>* le document en entier * | 1-16 | |
| A | PATENT ABSTRACTS OF JAPAN<br>vol. 10, no. 131 (C-346)<br>& JP 60 255714 A (POLA KASEI KOGYO KK)<br>* abrégé * | 1-16 | **DOMAINES TECHNIQUES RECHERCHES (Int.Cl.7)**<br><br>A61K |

-/--

### RECHERCHE INCOMPLETE

La division de la recherche estime que la présente demande de brevet, ou une ou plusieurs revendications, ne sont pas conformes aux dispositions de la CBE au point qu'une recherche significative sur l'état de la technique ne peut être effectuée, ou seulement partiellement, au regard de ces revendications.

Revendications ayant fait l'objet d'une recherche complète:

Revendications ayant fait l'objet d'une recherche incomplète:

Revendications n'ayant pas fait l'objet d'une recherche:

Raison pour la limitation de la recherche:

voir feuille supplémentaire C

| Lieu de la recherche | Date d'achèvement de la recherche | Examinateur |
|---|---|---|
| LA HAYE | 3 octobre 2001 | Fischer, J.P. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.................................................................
& : membre de la même famille, document correspondant

EPO FORM 1503 03.82 (P04C08)

Office européen
des brevets

**RECHERCHE INCOMPLETE
FEUILLE SUPPLEMENTAIRE C**

Numéro de la demande

EP 01 11 7660

Revendications ayant fait
l'objet de recherches complètes:
  aucune

Revendications ayant fait
l'objet de recherches incomplètes:
  1-16

Raison pour la limitation de la recherche:

Les revendications 1-16 présentes ont trait à une très grande variété de composés et de compositions. En effet les expressions "huile hydrocarbonée volatile constituée uniquement d'atomes de carbone et d'atomes d'hydrogène" et "cire synthétique fonctionnalisée portant au moins une fonction hydroxyle ou carboxyle" représentent un nombre très important de composés différents pouvant répondre à ces définitions. Un fondement au sens de L'Article 84 CBE et/ou un exposé au sens de l'Article 83 CBE ne peut cependant être trouvé que pour un nombre très restreint de ces produits revendiqués. Dans le cas présent, les revendications manquent à un tel point de fondement et l'exposé de l'invention dans la description est si limité q'une recherche significative couvrant tout le spectre revendiqué est impossible.

D'autre part les revendications 1-16 ont trait à un produit défini au moyen des paramètres suivants:

-le point de fusion de la cire synthétique est compris entre 75°c et 120°c.

-la cire est également définie par ses paramètres de solubilité de Hansen.

Le manque de clarté découlant de l'utilisation de ces paramètres est tel qu'une recherche significative et complète est impossible.

Par conséquent, la recherche a été limitée aux parties des revendications dont l'objet apparait être clair, fondé, et suffisamment exposé, à savoir les exemples et les composés cités dans la description et les revendications et dans l'esprit général de l'invention.

**Office européen des brevets**

**RAPPORT PARTIEL DE RECHERCHE EUROPEENNE**

Numero de la demande

EP 01 11 7660

| | DOCUMENTS CONSIDERES COMME PERTINENTS | | CLASSEMENT DE LA DEMANDE (Int.Cl.7) |
|---|---|---|---|
| Catégorie | Citation du document avec indication, en cas de besoin, des parties pertinentes | Revendication concernée | |
| A,P | FR 2 772 601 A (L'OREAL) 25 juin 1999 (1999-06-25) * le document en entier * ----- | 1-16 | |
| | | | **DOMAINES TECHNIQUES RECHERCHES** (Int.Cl.7) |

EPO FORM 1503 03.82 (P04C11)

**ANNEXE AU RAPPORT DE RECHERCHE EUROPEENNE
RELATIF A LA DEMANDE DE BREVET EUROPEEN NO.**

EP 01 11 7660

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

03-10-2001

| Document brevet cité au rapport de recherche | | Date de publication | Membre(s) de la famille de brevet(s) | | Date de publication |
|---|---|---|---|---|---|
| EP 850644 | A | 01-07-1998 | FR | 2757381 A1 | 26-06-1998 |
| | | | BR | 9706326 A | 04-05-1999 |
| | | | CA | 2223738 A1 | 24-06-1998 |
| | | | DE | 69700545 D1 | 28-10-1999 |
| | | | DE | 69700545 T2 | 30-12-1999 |
| | | | EP | 0850644 A1 | 01-07-1998 |
| | | | ES | 2139428 T3 | 01-02-2000 |
| | | | JP | 10194931 A | 28-07-1998 |
| | | | JP | 2001181140 A | 03-07-2001 |
| | | | KR | 273746 B1 | 15-12-2000 |
| | | | US | 6177091 B1 | 23-01-2001 |
| EP 847752 | A | 17-06-1998 | FR | 2756176 A1 | 29-05-1998 |
| | | | AT | 202470 T | 15-07-2001 |
| | | | BR | 9705926 A | 27-04-1999 |
| | | | CA | 2220033 A1 | 26-05-1998 |
| | | | DE | 69705379 D1 | 02-08-2001 |
| | | | EP | 1129685 A2 | 05-09-2001 |
| | | | EP | 0847752 A1 | 17-06-1998 |
| | | | JP | 3034490 B2 | 17-04-2000 |
| | | | JP | 10158118 A | 16-06-1998 |
| WO 9640044 | A | 19-12-1996 | CN | 1192133 A | 02-09-1998 |
| | | | EP | 0833601 A1 | 08-04-1998 |
| | | | JP | 11509177 T | 17-08-1999 |
| | | | WO | 9640044 A1 | 19-12-1996 |
| WO 9615761 | A | 30-05-1996 | WO | 9615761 A1 | 30-05-1996 |
| EP 709083 | A | 01-05-1996 | AU | 709441 B2 | 26-08-1999 |
| | | | AU | 3439195 A | 09-05-1996 |
| | | | BR | 9504533 A | 27-05-1997 |
| | | | CA | 2161285 A1 | 26-04-1996 |
| | | | CN | 1127631 A | 31-07-1996 |
| | | | EP | 0709083 A2 | 01-05-1996 |
| | | | FI | 955048 A | 26-04-1996 |
| | | | FR | 2726467 A1 | 10-05-1996 |
| | | | GB | 2294392 A ,B | 01-05-1996 |
| | | | GB | 2331013 A ,B | 12-05-1999 |
| | | | HK | 1013252 A1 | 12-05-2000 |
| | | | IL | 115693 A | 13-08-2000 |
| | | | JP | 8239316 A | 17-09-1996 |
| | | | NO | 954201 A | 26-04-1996 |
| | | | NZ | 280302 A | 20-12-1996 |
| | | | SG | 42788 A1 | 17-10-1997 |
| | | | US | 5985298 A | 16-11-1999 |

EPO FORM P0460

Pour tout renseignement concernant cette annexe : voir Journal Officiel de l'Office européen des brevets, No.12/82

**ANNEXE AU RAPPORT DE RECHERCHE EUROPEENNE
RELATIF A LA DEMANDE DE BREVET EUROPEEN NO.**

EP 01 11 7660

La présente annexe indique les membres de la famille de brevets relatifs aux documents brevets cités dans le rapport de recherche européenne visé ci-dessus.
Lesdits members sont contenus au fichier informatique de l'Officeeuropéen des brevets à la date du
Les renseignements fournis sont donnés à titre indicatif et n'engagent pas la responsabilité de l'Office européen des brevets.

03-10-2001

| Document brevet cité au rapport de recherche | | Date de publication | Membre(s) de la famille de brevet(s) | | Date de publication |
|---|---|---|---|---|---|
| EP 709083 | A | | US | 6274152 B1 | 14-08-2001 |
| | | | US | 5800816 A | 01-09-1998 |
| | | | US | 5965112 A | 12-10-1999 |
| | | | US | 5911974 A | 15-06-1999 |
| | | | ZA | 9509024 A | 06-08-1996 |
| JP 60255714 | A | 17-12-1985 | JP | 2087963 C | 02-09-1996 |
| | | | JP | 5074565 B | 18-10-1993 |
| FR 2772601 | A | 25-06-1999 | FR | 2772601 A1 | 25-06-1999 |
| | | | BR | 9805529 A | 11-04-2000 |
| | | | CN | 1225257 A | 11-08-1999 |
| | | | DE | 69800991 D1 | 02-08-2001 |
| | | | EP | 0930060 A1 | 21-07-1999 |
| | | | JP | 11246441 A | 14-09-1999 |
| | | | PL | 330457 A1 | 05-07-1999 |
| | | | US | 6254877 B1 | 03-07-2001 |

EPO FORM P0460

Pour tout renseignement concernant cette annexe : voir Journal Officiel de l'Office européen des brevets, No.12/82